# FASCICULE DE BREVET EUROPEEN

(11) **EP 2 897 984 B1**
(45) Date de publication et mention de la délivrance du brevet: **26.10.2016**
(21) Numéro de dépôt: 13765379.6
(22) Date de dépôt: 06.09.2013
(51) Int. Cl.: C08F 4/32, C08K 5/04, C08K 5/14, C08L 29/04, C08L 27/06, C08L 71/02

(54) **COMPOSITION D'EMULSION AQUEUSE DE PEROXYDE ORGANIQUE**
WÄSSRIGE EMULSIONZUSAMMENSETZUNG EINES ORGANISCHEN PEROXIDS
AQUEOUS EMULSION COMPOSITION COMPRISING AN ORGANIC PEROXIDE

(30) Priorité: 21.09.2012 FR 1258874
(43) Date de publication de la demande: 29.07.2015
(73) Titulaire: Arkema France, 92700 Colombes (FR)
(72) Inventeur: TARTARIN, Isabelle, F-69600 Oullins (FR); COCHET, Jacques, F-38150 Chanas (FR); BLUM, Albert, 89264 Weissenhorn (DE)
(74) Mandataire: Gavin, Pablo
(86) Numéro de dépôt international: PCT/FR2013/052060
(87) Numéro de publication internationale: WO 2014/044949

(56) Documents cités:
- EP-A1- 1 564 225
- WO-A2-2011/015567
- JP-A- 2001 064 312
- "Gosehnol General Grades K type", GOHSENOL.com , 20 août 2012 (2012-08-20), XP055064904, Extrait de l'Internet: URL:http://wayback.archive.org/web/2012082 0234421/http://www.gohsenol.com/doc_e/gnrl /gnrl_01/gnrl_05.shtml [extrait le 2013-06-03]

## Description

### Domaine de l'invention

L'invention a pour objet une composition aqueuse de peroxyde organique liquide à température de stockage, utilisable pour la polymérisation ou la copolymérisation de monomères éthyléniquement insaturés et en particulier de chlorure de vinyle. L'invention concerne plus particulièrement une composition aqueuse comprenant un émulsifiant à base d'acétate de polyvinyle ayant un haut degré d'hydrolyse et une faible viscosité en solution dans l'eau.

Des précautions particulières en termes de sécurité doivent être prises lors de la maintenance et de la manutention des peroxydes organiques. Les peroxydes organiques sont conditionnés sous forme d'émulsion aqueuse. La présence d'eau, en tant que fluide de transfert de chaleur, permet d'absorber et de dissiper l'énergie générée en cas d'éventuelles décompositions des peroxydes. De plus, ces émulsions comprennent un antigel, permettant un maintien de l'émulsion sous forme liquide, à des températures inférieures à -10 °C, généralement inférieures à -20 °C. Ces températures négatives permettent de prévenir la décomposition incontrôlée des peroxydes lors des opérations de stockage et de transport.

Une émulsion de peroxyde organique est constituée par des gouttelettes de peroxyde stabilisées par un émulsifiant. Au cours du temps, l'émulsion se déstabilise et la taille moyenne des gouttelettes de peroxyde augmente. L'augmentation de la taille des gouttelettes peut engendrer une séparation de phase. Selon les critères techniques minimums, une émulsion de peroxyde est considérée satisfaisante si la taille moyenne des gouttelettes ne dépasse pas 20 µm (micromètre). Une taille moyenne de gouttelettes inférieure à 10 µm, plus avantageusement inférieure à 5 µm, est généralement requise, de même qu'une taille maximale ne dépassant pas 20 µm.

En plus des considérations de sécurité dues à ce phénomène de déstabilisation, l'utilisation d'une émulsion de peroxyde organique non homogène en tant qu'amorceur de polymérisation dans une émulsion ou suspension de monomère vinylique peut produire une inhomogénéité dans le produit final. Cette inhomogénéité est généralement caractérisée par des particules de polymère mal gélifiées lors de la mise en oeuvre à l'état fondu (« fish eyes », grains durs). Or, La présence de grains durs opacifie le matériau polymère. Ces considérations de stabilité sont ainsi très importantes pour les applications où la transparence du produit final est impérative, notamment pour les applications médicales.

Ainsi, les gouttelettes de peroxyde (par agglomération du ou des peroxydes présents dans l'émulsion, en particulier après un certain laps de temps) d'une émulsion de peroxyde organique doivent avoir une taille moyenne faible, une distribution de taille homogène et monomodale, et être stables dans le temps. En particulier le diamètre maximal de ces gouttelettes ne devra pas dépasser 20 µm. En effet, des phénomènes d'agglomération ou de grossissement de gouttelettes de peroxyde peuvent conduire à une démixtion totale ou partielle de l'émulsion.

Les étapes de déchargement de l'émulsion dans des silos intermédiaires de stockage, de pompage et d'introduction d'une émulsion de peroxyde dans un réacteur de polymérisation sont des étapes importantes pour la qualité du polymère obtenu et la fiabilité du procédé de polymérisation. Ces étapes de manutention doivent être effectuées le plus rapidement possible. Pour se faire, il est crucial que l'émulsion de peroxyde présente une viscosité faible de sorte que l'écoulement de l'émulsion soit facilité au maximum. A une température donnée, la viscosité de ce type d'émulsion varie en particulier en fonction de la vitesse de cisaillement. Elle diminue lorsque la vitesse de cisaillement augmente et se stabilise pour des valeurs de vitesse généralement supérieures à 100 s⁻¹. Ainsi, une émulsion de peroxyde organique doit avoir une viscosité dynamique maximale de 1000 mPa.s (milliPascal seconde) à basse température, typiquement de l'ordre de -10 °C pour une vitesse de cisaillement de 100 s⁻¹. Les mesures de viscosité dynamique sont réalisées à l'aide de cylindres coaxiaux qui créent le cisaillement, par exemple selon la norme DIN 53019.

Or, l'homme du métier sait que, pour ce type d'émulsion, chercher à diminuer la taille des gouttelettes contribue à augmenter la viscosité (Voir paragraphe 1.4 de l'article de JP Canselier et M.Poux, « Procédés d'émulsification - Mécanisme de formation des émulsions » Techniques de l'Ingénieur J2 152, pp 1-12, publication du 10 juin 2004).

Ainsi, atteindre ces deux objectifs principaux simultanément est une difficulté importante pour l'homme du métier en raison des choix antinomiques qu'il est amené à envisager.

### Etat de l'art

L'utilisation d'acétate de polyvinyle (PVA) partiellement hydrolysé est largement décrite dans la littérature comme agent colloïde pour la stabilisation d'émulsion de peroxyde organique, tel que dans les documents EP0032757 et US3988261.

Un PVA non hydrolysé est insoluble dans l'eau. Le document WO 99/05101 divulgue l'utilisation de PVA ayant un degré d'hydrolyse compris entre 45 % et 68 % pour des émulsions aqueuses de peroxyesters. Ce document précise que des PVA ayant un degré d'hydrolyse supérieur à 68 %, produisent des émulsions trop visqueuses, non stable, dont la taille des gouttelettes varie de manière trop importante après un certain temps de stockage, ne pouvant prévenir les risques engendrés lors de la décomposition des peroxydes, inadaptées aux applications susvisées.

Le document WO 03/095500 divulgue l'utilisation de PVA comme colloïde protecteur, pour des émulsions aqueuses de peroxydicarbonates ou diacyles, ayant un degré d'hydrolyse dans la gamme large comprise entre 45 % et 80 %, plus précisément, tel que divulgué dans les exemples, autour d'une valeur du degré d'hydrolyse de 65 %.

Ainsi, l'état de l'art enseigne que, d'une part, dans une émulsion aqueuse de peroxyde organique, l'utilisation d'acétates de polyvinyle, ayant un degré d'hydrolyse inférieur à un maximum de 70 %, produisent des émulsions de peroxyesters, peroxydicarbonates et peroxyde de diacyles satisfaisant aux conditions drastiques de viscosité et de stabilité impérative à la manutention de telles émulsions et que d'autre part au-delà de cette limite quant au taux d'hydrolyse du PVA, l'utilisation de ce dernier n'est en aucun cas appropriée dans une telle émulsion.

### Brève description de l'invention

De façon surprenante, la demanderesse a découvert, en contradiction avec les enseignements de l'art antérieur, que des émulsifiants à base d'acétate de polyvinyle, ayant un haut degré d'hydrolyse, stabilisent des émulsions de peroxydes et répondent aux conditions requises concernant la taille des gouttelettes de peroxyde et la viscosité de l'émulsion. Ces caractéristiques sont remplies lors de la combinaison bien particulière de caractéristiques propres à l'acétate de polyvinyle, à savoir un haut degré d'hydrolyse avec une viscosité intrinsèque très faible.

La présente invention concerne ainsi, une composition d'émulsion aqueuse de peroxyde organique, comprenant:
- de 10 à 65 % en poids d'un ou plusieurs peroxydes organiques,
- de 2 à 25 % en poids d'au moins un agent antigel,
- de 0,01 à 10 % en poids d'au moins un agent émulsifiant,
- éventuellement au moins un additif,
- de l'eau, dont la quantité est déterminée de manière à former le reste de la composition totale (100 %),
caractérisée en ce que l'agent émulsifiant est un agent colloïde consistant en un acétate de polyvinyle ayant un degré d'hydrolyse supérieur à 80 % et une viscosité, mesurée en solution dans l'eau à 4 % en poids à 20 °C, inférieure ou égale à 5 mPa.s, ladite viscosité est mesurée avec un viscosimètre Brookfield RVT, aiguille n°3, 20 tpm (tour par minute), selon la norme ISO 2555.

D'autres caractéristiques ou modes de réalisation de l'invention sont présentées ci-après :
- de préférence, la viscosité de l'acétate de polyvinyle partiellement hydrolysé, mesurée en solution dans l'eau à 4 % en poids à 20 °C, est inférieure ou égale à 3 mPa.s (toujours dans les mêmes conditions de mesure) ;
- selon une possibilité offerte par l'invention, l'acétate de polyvinyle partiellement hydrolysé est modifié dans ses groupements acétates par des sels métalliques, préférentiellement choisi parmi des carboxylates de sodium et sulfonates ;
- avantageusement, la composition selon l'invention comprend un second agent émulsifiant consistant en un surfactant non ionique de type acide gras éthoxylé tels que les acides gras mono, di ou triglycérides éthoxylés, les huiles végétales éthoxylées ; alcool gras éthoxylé ; copolymère à blocs comprenant au moins un bloc d'oxyde d'alkylène ;
- de préférence, ce second agent émulsifiant consiste en une huile de ricin éthoxylée ;
- avantageusement, le degré d'hydrolyse de l'acétate de polyvinyle est supérieur à 85%, plus préférentiellement compris entre 86 % et 89 % ;
- de préférence, le ou les peroxydes organiques sont choisis parmi les peroxyesters, les peroxydicarbonates et/ou les peroxydes de diacyles ;
- selon une particularité propre à l'invention, la composition présente une viscosité définie par un temps d'écoulement inférieur à 200 secondes, préférentiellement inférieur à 100 secondes, ledit temps d'écoulement étant mesuré à la température de 5 °C selon la norme DIN 53211. Pour plus de précision, la norme DIN 53211 est ici caractérisée par un diamètre de la coupe de viscosité de 4 mm ; la viscosité dynamique est inférieure à 1000 mPa.s et mesurée à -10 °C pour une vitesse de cisaillement de 100 s⁻¹, à l'aide d'un Viscotester Haake VT550, selon la norme DIN 53019.
- de même, selon une caractéristique propre à l'invention, la composition comprend une pluralité de gouttelettes formées de peroxyde en ce que la taille moyenne (d₅₀) desdites gouttelettes est inférieure à 10 µm (micromètre), de préférence inférieure à 5 µm, et la taille maximale (d₁₀₀) des gouttelettes est inférieure à 20 µm ;
- avantageusement, la composition selon l'invention comprend plus de 30 % en poids d'un ou plusieurs peroxydes organiques, préférentiellement plus de 45 % en poids ;
- avantageusement, l'acétate de polyvinyle est présent entre 1 % et 5 % en poids, ou entre 0,5 % et 3 %.

L'invention présente les avantages suivants et permet ainsi l'obtention:
- d'une émulsion présentant une taille moyenne de gouttelettes faible avec une distribution de tailles homogène et monomodale,
- d'une émulsion comprenant une taille moyenne de gouttelettes (d₅₀) inférieure à 10 µm après production ou au cours du stockage à -20 °C pendant au moins 12 semaines. La taille maximale (d₁₀₀) ne dépasse pas les 20 µm.
- d'une émulsion compatible avec la polymérisation de dérivés éthyléniquement insaturés et en particulier de monomères vinyliques tels que le monomère de chlorure de vinyle,
- d'une émulsion liquide, ayant une très faible viscosité autorisant un temps d'écoulement très court.

La présente invention se rapporte également à un procédé de préparation de la composition selon l'une quelconque des revendications précédentes, caractérisé en ce qu'il comprend les étapes successives de :
- dispersion de l'agent antigel, éventuellement au moins ledit additif, ainsi que l'agent colloïde dans de l'eau pour obtenir une phase aqueuse homogène, puis
- le peroxyde est ajouté à la phase aqueuse, et
- le mélange ainsi constitué est émulsionné au cours d'une étape d'émulsion à une température inférieure à 5 °C, de préférence inférieure à -5 °C.

Enfin, l'invention se rapporte à l'utilisation de la composition susvisée pour la polymérisation ou la copolymérisation de monomères éthyléniquement insaturés. De préférence, ces monomères éthyléniquement insaturés comprennent le chlorure de vinyle.

L'émulsion de peroxyde organique selon la présente invention peut être utilisée dans des applications telles que la polymérisation de monomères acryliques, les réactions de modifications de polymères, les réactions de réticulation, les réactions de polymérisation en masse et les procédés de cuisson comme utilisés dans les résines polyesters insaturées.

### Description détaillée de l'invention

L'invention concerne des compositions de peroxyde organique concentré en émulsion, le dit peroxyde organique étant présent à une concentration de 10 % à 65 %, préférentiellement supérieure à 30 % et plus préférentiellement supérieure à 45 %, en poids de l'émulsion et est choisi parmi les peroxyesters, les peroxydicarbonates et les peroxydes de diacyles.

Parmi les peroxyesters, les peroxydes préférés sont le α-cumyl peroxyneodécanoate, α-cumyl peroxyneoheptanoate, le 2,4,4 triméthylpentyl-2-peroxyneodécanoate, le 3-hydroxy-1,1 diméthylbutyl peroxyneodécanoate, le 3-hydroxy-1,1 diméthylbutyl peroxyneoheptanoate, le tert-amyl peroxypivalate, le tert-butyl peroxypivalate, le tert-butyl peroxyneoheptanoate, le 2,5-diméthyl-2,5 di (2-éthylhexanoyl peroxy) hexane, le tert-amyl peroxy 2-éthylhexanoate, le tert-butyl peroxy 2-éthylhexanoate, le -1,1,3,3 tétraméthyl butyl-peroxy-2 éthylhexanoate, le 3-hydroxy-1,1 diméthylbutylperoxy 2-éthylhexanoate, le tert-butyl peroxy isobutyrate et leurs mélanges.

Parmi les peroxydicarbonates, les peroxydes préférés sont le peroxydicarbonate de di-sec-butyl, le peroxydicarbonate de dibutyl, le peroxydicarbonate de diisopropyl, le peroxydicarbonate de di(2-éthylhexyl), le peroxydicarbonate de bis(3-methoxybutyl), le peroxydicarbonate de bis(isobutyl), le peroxydicarbonate de dineopentyl, le peroxydicarbonate de bis(1-methylheptyl), le peroxydicarbonate de bis[2-(2-methoxyethoxy)éthyl], le peroxydicarbonate de bis(3-methoxy-3-méthylbutyl), le peroxydicarbonate de bis(2-ethoxyéthyl) et leurs mélanges.

Parmi les peroxydes de diacyles, les peroxydes préférés sont le peroxyde de diisobutyroyl, le peroxyde de di(3,5,5-triméthylhexanoyl), le peroxyde de di(2-ethylhexanoyl), le peroxyde de di(2-ethylbutanoyl), ainsi que les peroxydes asymétriques tels que, le peroxyde de isobutyroyl octanoyl, le peroxyde de isobutyroyl decanoyl, le peroxyde de isobutyroyl lauroyl, le peroxyde de 2-ethylbutanoyl decanoyl, le peroxyde de 2-éthylhexanoyl lauroyl, et leurs mélanges.

Afin de pouvoir être stockée à des températures inférieures à -10 °C, préférentiellement inférieures à -20 °C, la composition selon l'invention comprend un antigel ou plus particulièrement un mélange d'antigels.

S'agissant de l'agent anti-gel, on peut citer par exemple les monools, diols et triols tels que le méthanol, l'éthanol, l'éthylène glycol, l'isopropanol, le n-propanol, le propane-1,2-diol, le propane-1,3-diol, le glycérol, le butan-1-ol, le butan-2-ol, le butan-1,3-diol et le butan-1,4-diol et leurs mélanges, ces mélanges comprenant au moins deux des agents anti-gel énumérés précédemment, l'un de type alcool léger et l'autre de type alcool lourd, avantageusement un mélange de méthanol et de propane-1,2-diol.

L'émulsifiant selon l'invention est un acétate de polyvinyle ayant un degré d'hydrolyse supérieur à 80 % et une viscosité en solution dans l'eau à 4 % en poids à 20°C inférieure à 10 mPa.s, préférentiellement inférieure à 5 mPa.s.

S'agissant de l'acétate de polyvinyle partiellement hydrolysé, il peut consister en l'Alcotex^{®} 8804, le Mowiol^{®} 3-85 ou le Gohseran^{®} L3266, tous bien connus de l'homme du métier.

On ne sort pas du cadre de l'invention lorsqu'un mélange d'acétate de polyvinyle partiellement hydrolysé selon la présente invention est utilisé comme émulsifiant.

Selon un mode de réalisation, l'émulsifiant selon l'invention, à savoir l'acétate de polyvinyle partiellement hydrolysé, est modifié dans ses groupements acétates par des sels métalliques, préférentiellement choisis parmi des carboxylates de sodium et sulfonates.

Un mélange d'acétate de polyvinyle non modifié avec un acétate de polyvinyle modifié selon la présente invention peut être utilisé en tant que mélange d'émulsifiant pour la stabilisation d'émulsion de peroxyde organique.

Les PVA modifiés dans leurs groupements acétates sont préférentiellement choisis parmi le Gohseran^{®} L 3266, bien connu de l'homme du métier.

Un second émulsifiant non ionique peut être utilisé en combinaison avec l'acétate de polyvinyle partiellement hydrolysé, modifié ou non, selon l'invention et consiste en un surfactant non ionique de type acide gras éthoxylé, dérivés de mono, di, ou tri glycérides éthoxylés, huiles végétales éthoxylées, alcools gras éthoxylés, copolymère à blocs comprenant au moins un bloc d'oxyde d'alkylène. Les huiles végétales éthoxylées sont en particulier l'huile de ricin éthoxylée (hydrogénée ou non) comprenant 20 à 40 moles d'oxyde d'éthylène par mole d'acide ricinoléïque, des exemples commerciaux sont en particulier les Remcopal^{®} 20, Remcopal^{®} R4097 et Remcopal^{®} RH4090, bien connus de l'homme du métier.

Selon l'invention le second émulsifiant, un surfactant non ionique de type huile de ricin éthoxylée est présent à une concentration dans l'émulsion comprise entre 0,01 à 3% en poids, préférentiellement comprise entre 0,5 et 2 % en poids.

L'émulsion selon l'invention peut également comprendre un ou plusieurs additifs destinés à procurer à la composition thermoplastique finale des propriétés/caractéristiques particulières. Ces additifs seront idéalement présents pour la polymérisation ou copolymérisation finale.

Ainsi, s'agissant de l'additif, il peut être choisi parmi les antioxydants ; les agents de protection UV ; les agents de mise en oeuvre, ayant pour fonction d'améliorer l'aspect final lors de sa mise en oeuvre, telles que les amides grasses, l'acide stéarique et ses sels, l'éthylène bis-stéaramide ou les polymères fluorés ; les agents anti-buée ; les agents anti-bloquants tels que la silice ou le talc ; les charges telles que le carbonate de calcium et les nanocharges comme par exemple les argiles ; les agents de couplage tels que les silanes ; les agents réticulants comme les peroxydes ; les agents antistatiques ; les agents nucléants ; les pigments ; les colorants ; les plastifiants ; les fluidifiants et les additifs retardateurs de flamme tels que les hydroxydes d'aluminium ou de magnésium.

L'émulsion aqueuse liquide de peroxyde organique de la présente invention peut éventuellement contenir des additifs incluant les agents ajusteurs de pH tels que les tampons phosphate et citrate, les agents chélatants, des biocides par exemple des fongicides, des antiozonants, des antioxidants, des antidegradants, des agents gonflants et des agents de démoulage. L'émulsion peut également contenir des additifs habituellement utilisés pour stabiliser le peroxyde organique ou retarder sa décomposition tels que des phlegmatisants (isododécane, huile minérale) et des hydroperoxydes.

Ces additifs peuvent être ajoutés dans les quantites habituellement utilisées et connues de l'homme de l'art. Ces additifs sont généralement utilisés dans des teneurs comprises entre 10 ppm et 10 000 ppm en poids par rapport au poids de polymère ou de copolymère final. Les plastifiants, les fluidifiants et les additifs retardateurs de flamme peuvent atteindre des quantités bien supérieures à 10 000 ppm.

L'invention concerne également un procédé de préparation de l'émulsion précédemment décrite, caractérisé en ce que l'agent antigel, éventuellement un ou des additifs ainsi qu'au moins un émulsifiant sont dispersés dans de l'eau pour obtenir une phase aqueuse homogène puis le peroxyde est ajouté à la dite phase aqueuse, le tout étant ensuite émulsionné au cours d'une étape d'émulsion à une température inférieure à 5 °C, de manière à limiter la dégradation prématurée du peroxyde et de préférence inférieure à -5 °Celsius.

Hormis les étapes successives particulières du procédé de préparation de la composition selon l'invention, la préparation de l'émulsion ne se distingue en rien des techniques et appareils bien connus de l'homme du métier. La température à laquelle on prépare l'émulsion n'est pas critique mais elle doit être suffisamment réduite pour éviter un taux important de décomposition dont le résultat serait une perte du titre. La température choisie dépend essentiellement du ou des peroxydes organiques. Par ailleurs, pour préparer les émulsions aqueuses, on utilise classiquement de l'eau désionisée ou de l'eau distillée.

Le procédé de préparation comporte une étape d'émulsion avec un mélangeur à fort taux de cisaillement pour diviser et/ou homogénéiser au mieux le peroxyde dans la phase aqueuse. A titre d'exemple, on pourra citer des agitateurs à pâles et à ancre à rotation mécanique, les agitateurs à hélice, c'est-à-dire un ou plusieurs agitateurs montés sur un arbre commun, les agitateurs à turbine, c'est-à-dire ceux comportant des chicanes fixes sur la cuve mélangeuse ou en position adjacente aux organes agitateurs. On peut également utiliser des broyeurs colloïdaux et des homogénéisateurs. Selon une caractéristique de réalisation, le procédé selon l'invention est caractérisé en ce que l'on utilise un mélangeur ultrasonique ou un mélangeur rotor-stator.

Suite à la préparation de l'émulsion selon l'invention, les étapes de pompage et d'introduction des émulsions dans un réacteur de polymérisation doivent être effectuées le plus rapidement possible. Les émulsions de peroxyde doivent avoir une viscosité faible.

Ainsi, les émulsions de peroxydes organiques selon l'invention présentent une gamme de viscosité dynamique à -10 °C, 100 s⁻¹, inférieure à 1000 mPa.s, préférentiellement inférieure à 700 mPa.s juste après production. Les mesures de viscosité sont mesurées par exemples selon la norme DIN 53019 avec un appareil de type Viscotester Haake VT550, à -10 °C et pour une vitesse de cisaillement de 100 s⁻¹.

Leur coulabilité ou temps d'écoulement mesuré par une technique de coupe consistométrique est inférieure à 200 secondes, préférentiellement inférieure à 100 secondes (norme DIN 53211, effectuée avec un diamètre de la coupe de diamètre 4 mm et une température de 5 °C).

Les étapes ultérieures de polymérisation ou de copolymérisation ne sont, dans le cadre de la présente invention, pas différentes de celle de l'art antérieur. La polymérisation du monomère de chlorure de vinyle se fait en suspension à une température d'initiation comprise entre 45 et 70 °C.

L'invention a également trait à l'utilisation de l'émulsion définie ci-dessus pour la polymérisation ou la copolymérisation de monomères éthyléniquement insaturés. Un homopolymère est obtenu par polymérisation lorsqu'un seul monomère éthyléniquement insaturé est polymérisé. Un copolymère est obtenu par polymérisation lorsqu'au moins deux monomères éthyléniquement insaturés sont polymérisés. Il est entendu que les monomères sont capables de polymériser les uns avec les autres.

A titre de monomère éthyléniquement insaturé, on peut citer, les acrylates, esters vinyliques, monomère halogénure de vinyle, éthers vinyliques, composés vinyliques aromatiques tels que le styrène, le butadiène, et préférentiellement le chlorure de vinyle.

### Obtention des formulations des compositions testées :

Les PVA comparatifs, numérotés 1, 2, 3, 5, 7 et 8, ainsi que les PVA selon l'invention, numérotés 4, 6 et 9, servent d'émulsifiant aux émulsions 1 à 16 (les émulsions 11 à 16 utilisent les PVA précédemment numérotés 4, 6 et 9) et sont préparées selon le même mode opératoire.

La phase aqueuse contenant le PVA, l'agent antigel et l'eau est agitée entre 500 et 1000 tpm (tour par minute) et maintenue à une température de -5 °C (Celsius). Le peroxyde organique est ajouté progressivement dans le réacteur contenant ce mélange eau/PVA/antigel. L'agitation est maintenue pendant trois minutes à 2000 tpm. L'ensemble est ensuite fortement agité à l'aide d'un appareil de type rotor-stator « Ultraturrax type S-25N 18G » pendant deux minutes à 9500 tpm, puis sous agitation à l'aide d'une pâle à 1000 tpm pendant une minute. Chaque émulsion se fait sur 200 grammes au total.

### Tests réalisés :

Les mesures de viscosité dynamique sont réalisées à l'aide d'un viscosimètre de type « Viscotester Haake VT550 ». Le dispositif de mesure est le « SV-DIN 53019 », se référant à la norme DIN 53019. La mesure s'effectue à l'aide de cylindres coaxiaux qui créent le cisaillement. Entre 5 à 10 ml (millilitre) d'émulsion est introduite dans la chambre de mesure maintenue à -10 °C. Les valeurs données dans les exemples ci-dessous correspondant à une vitesse de cisaillement de 100 s⁻¹ et sont exprimées en mPa.s. La précision de la mesure est de ± 10 % de la valeur indiquée.

Les mesures de temps d'écoulement sont réalisées à l'aide de coupes consistométriques de type DIN 53211 (diamètre de la coupe de viscosité : 4 mm). La mesure se fait sur 100 g d'émulsion après conditionnement à une température de +5 °C. Les mesures de temps d'écoulement sont exprimées en secondes et la précision est de ±10 % de la valeur indiquée.

La taille des gouttelettes (d₁₀₀ et d₅₀) est déterminée par des moyens conventionnels en utilisant la technique de diffraction de la lumière. Le terme d₁₀₀ correspond au diamètre tel que 100% du volume de l'échantillon de gouttelettes de peroxyde organique dans l'émulsion aqueuse a un diamètre inférieur à d₁₀₀ et le terme d₅₀ correspond au diamètre moyen tel que 50% du volume des gouttelettes de peroxyde organique dans l'émulsion aqueuse a un diamètre inférieur à d₅₀. Les mesures sont faites en utilisant un dispositif de Malvern Master Sizer 2000^{®} à température ambiante. Les tailles de gouttelettes d₅₀ ou d₁₀₀ sont donnés à ± 0,5 µm.

### Matières premières des compositions testées :

Principalement deux types/familles d'émulsions ont été préparées pour réaliser les tests permettant de caractériser les compositions, selon l'art antérieur et selon l'invention.

La première émulsion consiste en du di (2-éthyle hexyle peroxydicarbonate) à 60% en poids et comprend:
- un système antigel qui est un mélange d'alcools de ratio massique 20/80 de propylène glycol/méthanol, de concentration globale 14 % ;
- un ratio eau/antigel de 64/36 en poids ;
- un taux de di (2-éthyle hexyle peroxydicarbonate) de 60 % en poids. Le di (2-éthyle hexyle peroxydicarbonate) est le Luperox^{®} 223 de la société Arkema de pureté 97 % ;
- un taux de polyvinyle acétate partiellement hydrolysé (PVA) de 1.2 % massique ;
- le complément est de l'eau distillée.

La seconde émulsion consiste en du *tert.*-butyl peroxyneodécanoate à 50 % en poids et comprend :
- un système antigel qui est un mélange d'alcools de ratio massique 40/60 de propylène glycol/méthanol, de concentration globale 16 % ;
- un ratio eau/antigel de 67/33 en poids ;
- un taux de tert butyl peroxyneodécanoate de 50 % en poids. Le tert-butyl peroxyneodécanoate est le Luperox^{®} 10 d'Arkema de pureté 97 % ;
- un taux de polyvinyle acétate partiellement hydrolysé (PVA) de 1.2 % massique ;
- le complément est de l'eau distillée.

### Caractérisations des PVA :

Les caractéristiques des PVA comparatifs et selon l'invention sont présentés dans le tableau 1 :

**TABLEAU 1**

| | Degré de polymérisation | Viscosité (mPa.s) | Degré d'hydrolyse (%) |
|---|---|---|---|
| PVA 1 | 800 | 5.6-6.6 | 72-73 |
| PVA 2 | 1630 | 36-42 | 78.5-81.5 |
| PVA 3 | 2560 | 44-52 | 78.5-81.5 |
| PVA 4 | **360** | **3.5-4.5** | **88** |
| PVA 5 | 2440 | 45-49 | 86.7-88.7 |
| PVA 6 | **300** | 3 | **85-90** |
| PVA 7 | Non connu | 6-7 | 86-90 |
| PVA 8 | 1700 | 20-26 | 85-90 |
| PVA 9 | **Non connu** | 2.5 | **87** |
| PVA 10 | 1100 | 2.2 | 42-45 |

La viscosité est mesurée à 4% dans l'eau à 20 °C (viscosité Brookfield RVT, aiguille n°3, 20 tpm).

Le PVA 9 est un PVA modifié par des fonctions sulfonates.

On constate bien que seuls les PVA n°4, n°6 et n°9 correspondent aux critères (faible viscosité & taux d'hydrolyse élevé) définis dans l'invention. Pour plus de facilité de lecture, dans le tableau comme dans les tableaux suivants, les PVA ou les émulsions selon l'invention sont présentés en caractère gras.

Un second émulsifiant de type surfactant non ionique de type huile de ricin éthoxylée de type Remcopal^{®} 20 (R20), est ajouté à 1% en poids dans les émulsions 10 (avec du PVA selon l'invention, plus précisément celui n°4 du tableau ci-dessus), 11 (avec du PVA selon l'invention, plus précisément celui n°6 du tableau ci-dessus), 15 (avec du PVA selon l'invention, plus précisément celui n°6 du tableau ci-dessus).

### Emulsions :

Les émulsions 1 à 11 et 16 correspondent à des émulsions de peroxyde de di(2-éthyle hexyle peroxydicarbonate), les émulsions 12 à 15 correspondent à des émulsions de peroxyde de *tert*.-butyl peroxyneodécanoate, et sont caractérisées dans les tableaux 2 et 3 :

**TABLEAU 2**

| | Emulsions 1-11, 16 | Emulsions 12-15 |
|---|---|---|
| di(2-éthyle hexyle peroxydicarbonate), % | 60,0 | |
| tert butyl peroxyneodécanoate, % | | 50,0 |
| méthanol, % | 11.2 | 9.6 |
| propylène glycol, % | 2.8 | 6.4 |
| tensioactif, % | 1,2 | 1,2 |
| eau, % | 24,8 | 32.8 |

**TABLEAU 3**

| | d₅₀ (µm) | d₁₀₀ (µm) | Viscosité (mPa.s) | Temps d'écoulement (s) |
|---|---|---|---|---|
| Emulsion 1 (PVA1) | 4 | 12 | 1100 | 145 |
| Emulsion 2 (PVA 2) | 7.1 | 112 | 2200 | > 300 |
| Emulsion 3 (PVA 3) | 7.4 | 141 | 1650 | > 300 |
| Emulsion 4 (PVA 4) | **3.9** | **13.3** | **790** | **92** |
| Emulsion 5 (PVA 5) | 8.6 | 100 | 3090 | > 300 |
| Emulsion 6 (PVA 6) | **3.4** | **12.6** | **670** | **72** |
| Emulsion 7 (PVA 7) | 4.1 | 19.9 | 1020 | 133 |
| Emulsion 8 (PVA 8) | 5.2 | 30.2 | 3250 | >300 |
| Emulsion 9 (PVA 9) | **2.7** | **7.6** | **580** | **66** |
| Emulsion 10 (PVA 4/R20) | **1.8** | **4.0** | **580** | **83** |
| Emulsion 11 (PVA 6/R20) | **1.8** | **4.0** | **530** | **63** |
| Emulsion 12 (PVA 1) | 3.3 | 8.7 | 608 | 64 |
| Emulsion 13 (PVA 9) | **3.3** | **8.7** | **190** | **26** |
| Emulsion 14 (PVA 6) | **3.8** | **11.1** | **363** | **38** |
| Emulsion 15 (PVA 6/R20) | **2.2** | **5.0** | **297** | **37** |
| Emulsion 16 (PVA 10) | 4.0 | 12.6 | 570 | 67 |

On notera qu'ici, les émulsions n° 10, 11, 13, 14 et 15 sont conformes à l'invention.

Des PVA à très fortes viscosités ne permettent pas d'obtenir des émulsions fluides, avec des temps d'écoulement courts et de tailles de gouttelettes suffisamment fines.

Plus la viscosité du PVA est faible et inférieure à 5 mPa.s, plus l'émulsion répond aux critères de performance en termes de fluidité.

Les viscosités des émulsions comprenant un surfactant non ionique de type huile de ricin éthoxylée (Remcopal^{®} 20 ou noté R20 ci-dessus), sont plus faible que celles contenant uniquement un PVA. En effet, l'ajout d'un surfactant non ionique tels qu'une huile de ricin éthoxylée de type Remcopal^{®} 20 à un émulsifiant PVA selon l'invention contribue à diminuer la viscosité de l'émulsion correspondante. Ainsi, la viscosité de l'émulsion obtenue est alors proche de celle réalisée avec un PVA modifié, par exemple par des fonctions sulfonates. En outre, en plus de diminuer la viscosité, l'ajout d'un second émulsifiant selon l'invention contribue à réduire la taille moyenne des gouttelettes de peroxyde organique.

D'une manière générale, une émulsion de peroxyester concentré à 50 % massique est légèrement plus fluide qu'une émulsion de peroxydicarbonate concentrée à 60 % massique. Par contre, les émulsions stabilisées par les PVA selon l'invention, sont plus fluides que celles comprenant un PVA comparatif (selon l'art antérieur). Dans le cas des peresters, l'ajout d'un surfactant non ionique de type huile de ricin éthoxylée. contribue, de nouveau, à diminuer la viscosité de l'émulsion tout en abaissant la taille des gouttelettes. Il doit être noté que les tests présentés ici n'incluent pas les peroxydes de diacyles mais les résultats obtenus sur les peroxyesters et les peroxydicarbonates permettent d'envisager des résultats similaires avec les peroxydes de diacyles. Les peroxydes de diacyles ont en effet fait l'objet de tests préliminaires aussi satisfaisants que ceux obtenus avec les peroxyesters et les peroxydicarbonates.

Les émulsions réalisées avec différents PVA comparatifs et selon l'invention sont stockées à une température de -20 °C pendant plusieurs mois. Les caractéristiques des différentes émulsions sont mesurées après différents temps de stockage. Les résultats sont présentés dans le tableau 4:

**TABLEAU 4**

| | Emulsion 1 (PVA1) | **Emulsion 6 (PVA 6)** | **Emulsion 11 (PVA 6/R20)** | **Emulsion 9 (PVA 9)** | Emulsion 16 (PVA 10) |
|---|---|---|---|---|---|
| d₅₀ (µm) | | | | | |
| t = 0 | 3.3 | 3.4 | 1.8 | 2.7 | 4.0 |
| t = 4 semaines | 3.6 | / | / | 3.1 | 4.2 |
| t = 8 semaines | 3.7 | 3.8 | 2.2 | 3.2 | 4.2 |
| t = 12 semaines | 3.8 | 4 | 2.3 | 3.3 | 4.4 |
| d₁₀₀ (µm) | | | | | |
| t = 0 | 10.0 | 12.6 | 4.0 | 7.6 | 12.6 |
| t = 4 semaines | 10.0 | / | / | 8.7 | 13.2 |
| t = 8 semaines | 10.0 | 14.8 | 5.8 | 8.7 | 15.1 |
| t = 12 semaines | 13.2 | 15.1 | 5.8 | 8.7 | 15.1 |
| Viscosité (mPa.s) | | | | | |
| t = 0 | 1100 | 670 | 530 | 580 | 570 |
| t = 4 semaines | 1180 | / | / | / | 560 |
| t = 8 semaines | 1170 | 730 | 430 | 500 | 630 |
| t = 12 semaines | 1170 | 750 | 400 | / | 670 |
| Temps d'écoulement (s) | | | | | |
| t = 0 | 145 | 72 | 63 | 66 | 67 |
| t = 4 semaines | 170 | / | / | 56 | 70 |
| t = 8 semaines | 171 | 90 | 59 | 52 | 77 |
| t = 12 semaines | 198 | 95 | 57 | / | 85 |

Les émulsions comprenant un PVA selon l'invention permettent de conserver les bonnes propriétés de l'émulsion après au minimum 12 semaines de stockage. Les viscosités des émulsions restent très fluides et inférieures à 1000 mPa.s (mesurées selon la norme DIN 53019 avec un Viscotester Haake VT550, à -10 °C et pour une vitesse de cisaillement de 100 s⁻¹), les temps d'écoulement restent inférieurs à 150 secondes et préférentiellement 100 secondes et les tailles maximales d₁₀₀ ne dépassent pas 20 µm (micromètre).

De manière générale, seules les compositions d'émulsion aqueuse de peroxyde selon l'invention permettent de résoudre les deux problèmes techniques majeurs, à savoir le grossissement des gouttelettes de peroxydes dans le temps ou autrement dit la stabilité de l'émulsion ainsi que la viscosité à froid de l'émulsion souvent trop élevée, entrainant notamment des temps d'écoulement non satisfaisant.

## Revendications

1. Composition d'émulsion aqueuse de peroxyde organique, comprenant:
- de 10 à 65 % en poids d'un ou plusieurs peroxydes organiques,
- de 2 à 25 % en poids d'au moins un agent antigel,
- de 0,01 à 10 % en poids d'au moins un agent émulsifiant,
- éventuellement au moins un additif,
- de l'eau, dont la quantité est déterminée de manière à former le reste de la composition totale (100 %),
**caractérisée en ce que** l'agent émulsifiant est un agent colloïde consistant en un acétate de polyvinyle ayant un degré d'hydrolyse supérieur à 80 % et une viscosité, mesurée en solution dans l'eau à 4 % en poids à 20 °C, inférieure ou égale à 3 mPa.s, ladite viscosité est mesurée avec un viscosimètre Brookfield RVT, aiguille n°3, 20 tpm, selon la norme ISO 2555.

2. Composition selon la revendication 1, **caractérisée en ce que** l'acétate de polyvinyle partiellement hydrolysé est modifié dans ses groupements acétates par des sels métalliques, préférentiellement choisi parmi des carboxylates de sodium et sulfonates.

3. Composition selon l'une quelconque des revendications précédentes, **caractérisée en ce qu'**elle comprend un second agent émulsifiant consistant en un surfactant non ionique de type acide gras éthoxylé tels que les acides gras mono, di ou triglycérides éthoxylés, les huiles végétales éthoxylées ; alcool gras éthoxylé ; copolymère à blocs comprenant au moins un bloc d'oxyde d'alkylène.

4. Composition selon la revendication 3, **caractérisée en ce que** le second agent émulsifiant consiste en une huile de ricin éthoxylée.

5. Composition selon l'une quelconque des revendications précédentes, **caractérisée en ce que** le degré d'hydrolyse de l'acétate de polyvinyle est supérieur à 85%, plus préférentiellement compris entre 86 % et 89 %.

6. Composition selon l'une quelconque des revendications précédentes, **caractérisée en ce que** le ou les peroxydes organiques sont choisis parmi les peroxyesters, les peroxydicarbonates et/ou les peroxydes de diacyles.

7. Composition selon l'une quelconques des revendications précédentes, **caractérisée en ce qu'**elle présente une viscosité définie par un temps d'écoulement inférieur à 200 secondes, préférentiellement inférieur à 100 secondes, ledit temps d'écoulement étant mesuré à la température de 5 °C selon la norme DIN 53211 avec un diamètre de la coupe de 4 mm.

8. Composition selon l'une quelconques des revendications précédentes, **caractérisée en ce qu'**elle comprend une pluralité de gouttelettes formées de peroxyde en ce que la taille moyenne (d₅₀) desdites gouttelettes est inférieure à 10 µm (micromètre), de préférence inférieure à 5 µm, et la taille maximale (d₁₀₀) des gouttelettes est inférieure à 20 µm.

9. Composition selon l'une quelconque des revendications précédentes, **caractérisée en ce qu'**elle comprend plus de 30 % en poids d'un ou plusieurs peroxydes organiques, préférentiellement plus de 45 % en poids.

10. Composition selon l'une quelconque des revendications précédentes, **caractérisée en ce que** l'acétate de polyvinyle est présent entre 1 % et 5 % en poids.

11. Composition selon l'une quelconque des revendications 1 à 9, **caractérisée en ce que** l'acétate de polyvinyle est présent entre 0,5 % et 3% en poids.

12. Procédé de préparation de la composition selon l'une quelconque des revendications précédentes, **caractérisé en ce qu'**il comprend les étapes successives de :
- dispersion de l'agent antigel, éventuellement au moins ledit additif, ainsi que l'agent colloïde dans de l'eau pour obtenir une phase aqueuse homogène, puis
- le peroxyde est ajouté à la phase aqueuse, et
- le mélange ainsi constitué est émulsionné au cours d'une étape d'émulsion à une température inférieure à 5 °C, de préférence inférieure à -5 °C.

13. Utilisation de la composition selon l'une des revendications 1 à 11 pour la polymérisation ou la copolymérisation de monomères éthyléniquement insaturés.

14. Utilisation de la composition selon la revendication 12, **caractérisée en ce que** les monomères éthyléniquement insaturés comprennent le chlorure de vinyle.

## Patentansprüche

1. Wässrige Emulsionszusammensetzung von organischem Peroxid, umfassend:
- 10 bis 65 Gew.-% eines oder mehrerer organischer Peroxide,
- 2 bis 25 Gew.-% mindestens eines Gefrierschutzmittels,
- 0,01 bis 10 Gew.-% mindestens eines Emulgators,
- gegebenenfalls mindestens ein Additiv,
- Wasser, dessen Menge so bemessen ist, dass sie den Rest der gesamten Zusammensetzung (100%) bildet, **dadurch gekennzeichnet, dass** es sich bei dem Emulgator um ein Kolloidmittel handelt, das aus einem Polyvinylacetat mit einem Hydrolysegrad von mehr als 80% und einer in 4 gew.-%iger Lösung in Wasser bei 20°C gemessenen Viskosität kleiner gleich 3 mPa.s besteht, wobei die Viskosität mit einem Brookfield-RVT-Viskosimeter, Spindel Nr. 3, 20 U/min, gemäß ISO-Norm 2555 gemessen wird.

2. Zusammensetzung nach Anspruch 1, **dadurch gekennzeichnet, dass** das teilhydrolysierte Polyvinylacetat in seinen Acetatgruppen durch Metallsalze, die vorzugsweise aus Natriumcarboxylaten und Sulfonaten ausgewählt sind, modifiziert ist.

3. Zusammensetzung nach einem der vorhergehenden Ansprüche, **dadurch gekennzeichnet, dass** sie einen zweiten Emulgator umfasst, der aus einem nichtionischen Tensid vom Typ ethoxylierte Fettsäure wie ethoxylierten Fettsäuremono-, Fettsäuredi- und Fettsäuretriglyceriden, ethoxylierten Pflanzenölen; ethoxylierter Fettalkohol oder Blockcopolymer mit mindestens einem Alkylenoxid-Block besteht.

4. Zusammensetzung nach Anspruch 3, **dadurch gekennzeichnet, dass** der zweite Emulgator aus einem ethoxylierten Ricinusöl besteht.

5. Zusammensetzung nach einem der vorhergehenden Ansprüche, **dadurch gekennzeichnet, dass** der Hydrolysegrad des Polyvinylacetats größer als 85% ist und weiter bevorzugt zwischen 86% und 89% liegt.

6. Zusammensetzung nach einem der vorhergehenden Ansprüche, **dadurch gekennzeichnet, dass** das bzw. die organischen Peroxide aus Peroxyestern, Peroxydicarbonaten und/oder Diacylperoxiden ausgewählt sind.

7. Zusammensetzung nach einem der vorhergehenden Ansprüche, **dadurch gekennzeichnet, dass** sie eine durch eine Auslaufzeit von weniger als 200 Sekunden, vorzugsweise weniger als 100 Sekunden, definierte Viskosität aufweist, wobei die Auslaufzeit bei der Temperatur von 5°C gemäß DIN-Norm 53211 mit einem Becherdurchmesser von 4 mm gemessen wird.

8. Zusammensetzung nach einem der vorhergehenden Ansprüche, **dadurch gekennzeichnet, dass** sie mehrere Tröpfchen aus Peroxid umfasst, dass die mittlere Größe (d₅₀) der Tröpfchen kleiner als 10 µm (Mikrometer), vorzugsweise kleiner als 5 µm, ist und die maximale Größe (d₁₀₀) der Tröpfchen kleiner als 20 µm ist.

9. Zusammensetzung nach einem der vorhergehenden Ansprüche, **dadurch gekennzeichnet, dass** sie mehr als 30 Gew.-% eines oder mehrerer organischer Peroxide, vorzugsweise mehr als 45 Gew.-%, umfasst.

10. Zusammensetzung nach einem der vorhergehenden Ansprüche, **dadurch gekennzeichnet, dass** das Polyvinylacetat in einer Menge zwischen 1 und 5 Gew.-% vorliegt.

11. Zusammensetzung nach einem der Ansprüche 1 bis 9, **dadurch gekennzeichnet, dass** das Polyvinylacetat in einer Menge zwischen 0,5 und 3 Gew.-% vorliegt.

12. Verfahren zur Herstellung der Zusammensetzung nach einem der vorhergehenden Ansprüche, **dadurch gekennzeichnet, dass** es folgende aufeinanderfolgende Schritte umfasst:
- Dispergieren des Gefrierschutzmittels, gegebenenfalls mindestens des Additivs sowie des Kolloidmittels in Wasser zum Erhalt einer homogenen wässrigen Phase, dann
- das Peroxid wird zu der wässrigen Phase gegeben, und
- die so gebildete Mischung wird im Lauf eines Emulgierungsschritts bei einer Temperatur von weniger als 5°C, vorzugsweise weniger als -5°C, emulgiert.

13. Verwendung der Zusammensetzung nach einem der Ansprüche 1 bis 11 zur Polymerisation oder Copolymerisation von ethylenisch ungesättigten Monomeren.

14. Verwendung der Zusammensetzung nach Anspruch 12, **dadurch gekennzeichnet, dass** die ethylenisch ungesättigten Monomeren Vinylchlorid umfassen.

## Claims

1. An aqueous organic peroxide emulsion composition comprising:
- from 10% to 65% by weight of one or more organic peroxides,
- from 2% to 25% by weight of at least one antifreeze agent,
- from 0.01% to 10% by weight of at least one emulsifying agent,
- optionally at least one additive,
- water, the amount of which is determined so as to form the remainder of the total composition (100%),
**characterized in that** the emulsifying agent is a colloid agent consisting of a polyvinyl acetate having a degree of hydrolysis of greater than 80% and a viscosity, measured in solution in water at 4% by weight at 20°C, of less than or equal to 3 mPa.s, said viscosity being measured with a Brookfield RVT viscometer, spindle No. 3, 20 rpm, according to the standard ISO 2555.

2. Composition according to claim 1, **characterized in that** the partially hydrolyzed polyvinyl acetate is modified in its acetate groups by metal salts, preferably chosen from sodium carboxylates and sulfonates.

3. Composition according to any one of the preceding claims, **characterized in that** it comprises a second emulsifying agent consisting of a nonionic surfactant of ethoxylated fatty acid type such as ethoxylated fatty acid mono-, di- or triglycerides or ethoxylated vegetable oils; ethoxylated fatty alcohol; a block copolymer comprising at least one alkylene oxide block type.

4. Composition according to claim 3, **characterized in that** the second emulsifying agent consists of an ethoxylated castor oil.

5. Composition according to any one of the preceding claims, **characterized in that** the degree of hydrolysis of the polyvinyl acetate is greater than 85%, more preferably between 86% and 89%.

6. Composition according to any one of the preceding claims, **characterized in that** the organic peroxide or peroxides are chosen from peroxyesters, peroxydicarbonates and/or diacyl peroxides.

7. Composition according to any one of the preceding claims, **characterized in that** it exhibits a viscosity defined by a flow time of less than 200 seconds, preferably of less than 100 seconds, said flow time being measured at the temperature of 5°C according to the standard DIN 53211 with a diameter of the cup of 4 mm.

8. Composition according to any one of the preceding claims, **characterized in that** it comprises a plurality of droplets formed of peroxide **in that** the mean size (d₅₀) of said droplets is less than 10 µm (micrometer), preferably less than 5 µm, and the maximum size (d₁₀₀) of the droplets is less than 20 µm.

9. Composition according to any one of the preceding claims, **characterized in that** it comprises more than 30% by weight of one or more organic peroxides, preferably more than 45% by weight.

10. Composition according to any one of the preceding claims, **characterized in that** the polyvinyl acetate is present at between 1% and 5% by weight.

11. Composition according to any one of claims 1 to 9, **characterized in that** the polyvinyl acetate is present at between 0.5% and 3%.

12. A process for the preparation of the composition according to any one of the preceding claims, **characterized in that** it comprises the successive stages of:
- dispersion of the antifreeze agent, optionally at least said additive and also the colloid agent in water, in order to obtain a homogeneous aqueous phase, then
- the peroxide is added to the aqueous phase, and
- the mixture thus formed is emulsified during an emulsification stage at a temperature of less than 5°C, preferably of less than -5°C.

13. The use of the composition as claimed in one of claims 1 to 11 in the polymerization or the copolymerization of ethylenically unsaturated monomers.

14. The use of the composition as claimed in claim 12, **characterized in that** the ethylenically unsaturated monomers comprise vinyl chloride.
